# EUROPEAN PATENT SPECIFICATION

(11) **EP 1 545 611 B1**
(45) Date of publication and mention of the grant of the patent: **09.11.2016**
(21) Application number: 03752016.0
(22) Date of filing: 05.09.2003
(51) Int. Cl.: C07K 16/18, A61K 39/00

(54) **METHOD OF TREATMENT OF ASTHMA USING ANTIBODIES TO COMPLEMENT COMPONENT C5**
VERFAHREN ZUR BEHANDLUNG VON ASTHMA MIT ANTIKÖRPERN ZUR KOMPLEMENTIERUNG VON KOMPONENTE C5
PROCEDE DE TRAITEMENT DE L'ASTHME METTANT EN OEUVRE DES ANTICORPS EN COMPLEMENT DU CONSTITUANT C5

(30) Priority: 06.09.2002 US 408571 P; 09.05.2003 US 469189 P
(43) Date of publication of application: 29.06.2005
(73) Proprietor: ALEXION PHARMACEUTICALS, INC., Cheshire, CT 06410 (US)
(72) Inventor: WANG, Yi, Orange, CT 06477 (US)
(74) Representative: D Young & Co LLP
(86) International application number: PCT/US2003/027808
(87) International publication number: WO 2004/022096

(56) References cited:
- EP-B1- 0 649 468
- US-B1- 6 316 502
- US-B1- 6 355 245
- KRUG NORBERT ET AL: "Complement factors C3a and C5a are increased in bronchoalveolar lavage fluid after segmental allergen provocation in subjects with asthma" AMERICAN JOURNAL OF RESPIRATORY AND CRITICAL CARE MEDICINE, vol. 164, no. 10 Part 1, 15 November 2001 (2001-11-15), pages 1841-1843, XP002363574 ISSN: 1073-449X
- ABE M ET AL: "Contribution of anaphylatoxin C5a to late airway responses after repeated exposure of antigen to allergic rats." JOURNAL OF IMMUNOLOGY (BALTIMORE, MD. : 1950) 15 OCT 2001, vol. 167, no. 8, 15 October 2001 (2001-10-15), pages 4651-4660, XP002363575 ISSN: 0022-1767
- LUKACS NICHOLAS W ET AL: "Complement-dependent immune complex-induced bronchial inflammation and hyperreactivity" AMERICAN JOURNAL OF PHYSIOLOGY, vol. 280, no. 3 Part 1, March 2001 (2001-03), pages L512-L518, XP002363576 ISSN: 0002-9513
- PENG TAO ET AL: "Role of C5 in the development of airway inflammation, airway hyperresponsiveness, and ongoing airway response." THE JOURNAL OF CLINICAL INVESTIGATION. JUN 2005, vol. 115, no. 6, June 2005 (2005-06), pages 1590-1600, XP002363577 ISSN: 0021-9738
- THOMAS T C ET AL: "Inhibition of complement activity by humanized anti-C5 antibody and single-chain Fv", MOLECULAR IMMUNOLOGY, PERGAMON, GB, vol. 33, no. 17-18, 1 January 1996 (1996-01-01), pages 1389-1401, XP002262113, ISSN: 0161-5890, DOI: 10.1016/S0161-5890(96)00078-8

## Description

### BACKGROUND

### TECHNICAL FIELD

This disclosure relates to a method of treating asthma using a compound which binds to or otherwise blocks the generation and/or activity of one or more complement components such as, for example, a complement-inhibiting antibody.

### DESCRIPTION OF THE RELATED ART

Asthma, bronchitis and emphysema are known collectively as Chronic Obstructive Pulmonary Diseases. These diseases are characterized as generalized airways obstruction, especially of small airways, associated with varying degrees of symptoms of chronic bronchitis, asthma, and emphysema. These diseases may often coexist in an individual, and it may be difficult to determine the primary cause of an airway obstruction. Airway obstruction is defined as an increased resistance to airflow during forced expiration. Obstruction of large airways may also occur in these diseases, particularly in asthma.

Asthma is a reversible obstructive pulmonary disorder caused by an airway hyper-responsiveness to specific and/ or non-specific stimuli. Asthmatic airway obstruction typically results from bronchospasms. Asthma may be triggered by a variety of causes such as allergic reactions, a secondary response to infections, industrial or occupational exposures, ingestion of certain chemicals or drugs, exercise, and vasculitis. Much of asthma's pathology can be attributed to mast cell degranulation. Mast cells will degranulate in response to various conditions such as, for example, classical IgE-antigen stimulation. It is believed that when the asthmatic, human or animal, inhales an allergenic substance, sensitized IgE antibodies trigger mast cell degranulation in the lung interstitium. The mast cell degranulation releases histamine, bradykinin, and slow-reacting substance of anaphylaxis (SRS-A) which includes the leukotrienes C, D and E, prostaglandins including PGF₂, PGF_{2α}, and PGD₂, and thromboxane A₂. The histamine then attaches to receptor sites in the larger bronchi, causing irritation, inflammation, and edema. The SRS-A attaches to receptor sites in the smaller bronchi, causing edema and attracting prostaglandins, which enhance the effects of histamine in the lungs. Histamine, in combination with prostaglandins, also stimulates excessive mucous secretion, narrowing the bronchial lumen further. When an asthmatic individual inhales, the narrowed bronchial lumen still expands slightly, allowing air to reach the alveoli. However, upon exertion to exhale, the increased thoracic pressure closes the bronchial lumen completely. Therefore, air can enter the lungs, but may not exit during an asthma attack. The ventilation in the alveoli is then inhibited by mucous collecting in the lung bases. In an effort to compensate for lowered alveolar ventilation, blood is shunted to other alveoli. Hypoxia, and in extreme cases, respiratory acidosis may result without medical intervention. In many cases, there are two phases to an allergic asthma attack, an early phase and a late phase which follows 4-6 hours after bronchial stimulation. The early phase includes the immediate inflammatory response including the reactions caused by the release of cellular mediators from mast cells (i.e., histamine). Late phase reactions develop over a period of hours and are characterized histologically by an early influx of polymorphonuclear leukocytes and fibrin deposition, later followed by infiltration of eosinophils. Increased levels of eosinophil-derived inflammatory mediators in plasma and BAL, including eosinophilic cationic protein and major basic protein, have been observed during the late phase reaction. Upregulation of TH2 -type cytokines (IL4, IL5 and IL 13) following allergen challenge has also been observed during the late phase. Thus, the cellular inflammatory response, in combination with released pro-inflammatory mediators (e.g., mmp9) and locally produced cytokines in the bronchial mucosa, play a central role in the late phase allergic inflammation and bronchoconstriction. Late phase reactions increase airway reactivity and lead to prolonged asthmatic exacerbations that may last from hours to days to months in some subjects. One of the residual effects of asthma reactions is this hyperresponsiveness of the airways to nonspecific stimuli.

Currently, the treatments for asthma are not always adequate and many have serious side effects. The general goals of drug therapy for asthma are prevention of bronchospasm and control of airway hyperreactivity or hyperresponsiveness, an indication of airway inflammation. It is very difficult to eliminate or prevent exposure to all allergens that may trigger an asthma attack. To prevent these attacks, most asthmatics are treated with various pharmacological agents, many of which have side effects.

In a study reported by Lukacs et al. (Lukacs at el, Am. J. Physiol Lung Cell Mol Physiol. 2001, vol. 280, pages L512-L518), anti-C5a antibodies were administered intratracheally togeher with anti-BSA antibody during the induction of immune complex mediated lung inflammation. Specifically, the Lukacs study uses a model of acute immune complex mediated tissue lung inflammation similar to reverse passive Arthus reaction in skin, with a brief AHR to intravenous challenges of methacholine. Animals did not develop chronic airway inflammation featured by eosinophilia; nor did they experience previous severe asthmatic attack after exposed to allergens as demonstrated in the current invention. The key feature of Lukacs study was the formation of immune complexes of BSA and anti-BSA locally along the airway, which activate the complement cascade and produced significantly amount of terminal complement components after intratracheally injected anti-BSA antibody into animals. The subsequent development of AHR to methacholine lasting up to four hours during the acute phase BSA-anti-BSA induced lung inflammation, which is in significantly contrast to the severe and long lasting AHR seen patients with asthma. It is reasonable to assume based on the Lukacs study that anti-C5a antibody neutralizes C5a produced locally in the airway and therefor prevents the development of harmful downstream events mediated by C5a such as recruiting and activating of inflammatory cells, stimulating the release of mediators and vascular leaky syndromes. The combination effects of blocking C5a mediated recruitment of inflammatory cells, release of mediators such as histamine, and the development of edema of airways that leads to the reduction of airway resistance and prevention of the development of AHR when animals were challenged with IV methacholine. This study may suggest the importance of C5a in development of AHR of immune complexes mediated lung inflammation. However, this study provides no basis to predict by if there is any direct and immediate effect of bronchial dilation by C5 inhibitors during an on-going asthmatic attack or late phase airway responses to allergens. Nor does this study involve the treatment of subjects that have established airway inflammation or subjects that have experienced previous asthmatic symptoms.

Abe et al. (J Immunol. 2001 Oct 15;167(8):4651-60) investigated the contribution of anaphylatoxin C5a to late airway responses after repeated exposure of antigen to allergic rats. Krug et al. (Am J Respir Crit Care Med. 2001 Nov 15;164:1841-3) discloses that complement factors C3a and C5a are increased in bronchoalveolar lavage fluid after segmental allergen provocation in subjects with asthma
Thomas et al. (Mol Immunol. 1996 Dec;33(17-18):1389-401) investigates the Inhibition of complement activity by humanized anti-C5 antibody and single-chain Fv.

### SUMMARY

According to a first aspect of the present invention there is provided an anti-C5 antibody for use in treating asthma in a subject susceptible to or having asthma, wherein the anti-C5 antibody is h5G1.1.

According to another aspect of the present invention there is provided an anti-C5 antibody for use in reducing the severity of an asthma attack in a subject having an asthma attack, wherein the anti-C5 antibody is h5G1.1.

According to another aspect of the present invention there is provided an anti-C5 antibody for use in reducing airway obstruction in a subject having asthma, wherein the anti-C5 antibody is h5G1.1

According to another aspect of the present invention there is provided a drug and an anti-C5 antibody in combination for use in treating a subject susceptible to or having asthma, wherein the anti-C5 antibody is h5G1.1 and wherein the drug is selected from the group consisting of steroids, anti-IgE antibodies, anti-IL-4 antibodies, anti-IL-5 antibodies, β2 adreno receptor agonists, leukotriene inhibitors, 5 Lipoxygenase inhibitors, PDE inhibitors, CD23 antagonists, IL-13 antagonists, cytokine release inhibitors, histamine H1 receptor antagonists, anti-histamines, and histamine release inhibitors.

There is described herein a treatment for asthma using a compound which binds to or otherwise blocks the generation and/or activity of one or more complement components or blocks the engagement of complement component receptors such as, for example, C5a receptors. The treatment therapy described herein includes the administration of a compound that inhibits the production and/or activity of at least one complement component. Suitable compounds include, for example, antibodies which bind to or otherwise block the generation and/or activity of one or more complement components, such as, for example, an antibody specific to complement component C5.

The complement-inhibiting compound can be administered prophylactically in known asthmatic individuals (such as those having established airway inflammation or a subject that has experienced previous asthmatic symptoms) to prevent, or help prevent asthma attacks. This prophylactic therapy can be administered via intravenous, aerosol, subcutaneous or intramuscular routes.

The complement-inhibiting compound can be administered as a therapeutic regimen to an individual experiencing an asthma attack. The regimen can be administered via intravenous, aerosol, subcutaneous or intramuscular routes.

A combination therapy may also be used that includes a complement-inhibiting compound in combination with a regimen of known asthma therapy, such as, for example, steroids, anti-IgE antibodies, anti-IL-4 antibodies, anti-IL-5 antibodies, β2 receptor agonists, leukotriene inhibitors, 5 Lipoxygenase inhibitors, b2 adreno receptor agonists, PDE inhibitors, IL 5 antagonists, CD23 antagonists, IL 13 antagonists, cytokine release inhibitors, histamine H1 receptor antagonists, anti-histamines and histamine release inhibitors. Suitable compounds of each class listed above as well as other asthma treatments are listed in Asthma Therapeutic: New Treatment Options and Emerging Drug Discovery Tasrgerts, Barnes, April 2003, Lead Discovery, http://www.leaddiscovery.co.uk/target-discovery/abstracts/dossier-asthma.html

A method of reducing inflammation in the lungs of asthma patients is also described herein. The methods include the step of administering to a subject having or susceptible to asthma a compound that reduces the release or production of inflammatory mediators (such as, for example, matrix metalloprotease 9 (mmp9 - also known as the 92-kDa type IV collagenase/gelatinase or gelatinase B), TGFβ, eosinophil granules, and the like) in the airways of the subject. The compound can act at the cellular level to reduce the production or release of the inflammatory mediator, can interact with the inflammatory mediator in a manner that interfaces with its activity, (such as, for example by preventing the conversion of pro-mmp-9 to the 83 kDa active form or can interact with an active form) of the inflammatory mediator to prevent the inflammatory effects associated therewith.

### BRIEF DESCRIPTION OF DRAWINGS

Figure 1 graphically shows the OVA-induced asthmatic reactions in normal BALB/c Mice.
Figure 2a shows the schedule and nature of antigen challenges and prophylactic treatment of normal BALB/c Mice.
Figure 2b shows a schedule of antigen challenge and prophylactic treatment in normal BALB/c Mice.
Figure 3 graphically summarizes the effects of the treatments shown in Figures 2a and 2b.
Figure 4a shows the schedule and nature of antigen challenges and IV or aerosol treatment of normal BALB/c Mice during an asthma attack.
Figure 4b depicts the protocol and results of induction of asthmatic attack in BALB/c Mice.
Figures 5a and 5b graphically summarize the effects of the aerosol treatments shown in Figure 4a.
Figures 6a and 6b graphically summarize the effects of the intravenous treatments shown in Figure 4a.
Figure 7 graphically depicts the systemic effects of various treatments on C5 activity for treatments shown in Figure 4a.
Figure 8 graphically shows the effect of various intravenous treatments on the total WBC count in BAL.
Figures 9 and 10 show that eosinophils were found to be the predominant inflammatory cells in BAL.
Figure 11 graphically shows the effect of various intravenous treatments on histamine and in BAL.
Figure 12 graphically shows the effect of various intravenous treatments on MMP-9 levels in BAL
Figure 13 graphically shows the effect of various intravenous treatments on TGFβ levels in BAL.
Figure 14 shows the schedule and nature of antigen challenges and cannulation and aerosol treatment of normal BALB/c Mice during an asthma attack.
Figure 15 shows lung resistance in asthmatic mice treated during an asthma attack with anti-C5, β2 receptor agonist or a combination thereof.

### DETAILED DESCRIPTION

The present disclosure is directed to a method of treating asthma in mammals. Specifically, the methods of treating asthma described herein involve using compounds which bind to or otherwise block the generation and/or activity of one or more complement components. The inhibition or blocking of the generation of complement components inhibits multiple factors involved in the broncho-constrictive responses in asthma. A specific class of such compounds which are particularly useful are antibodies specific to a human complement component, especially anti-C5 antibodies.

The complement system acts in conjunction with other immunological systems of the body to defend against intrusion of cellular and viral pathogens. There are at least 25 complement proteins, which are found as a complex collection of plasma proteins and membrane cofactors. The plasma proteins make up about 10% of the globulins in vertebrate serum. Complement components achieve their immune defensive functions by interacting in a series of intricate but precise enzymatic cleavage and membrane binding events. The resulting complement cascade leads to the production of products with opsonic, immunoregulatory, and lytic functions. A concise summary of the biologic activities associated with complement activation is provided, for example, in The Merck Manual, 16^{th} Edition.

The complement cascade progresses via the classical pathway or the alternative pathway. These pathways share many components, and while they differ in their initial steps, they converge and share the same "terminal complement" components (C5 through C9) responsible for the activation and destruction of target cells.

The classical complement pathway is typically initiated by antibody recognition of and binding to an antigenic site on a target cell. The alternative pathway is usually antibody independent, and can be initiated by certain molecules on pathogen surfaces. Additionally, the lectin pathway is typically initiated with binding of mannose-binding lectin (MBL) to high mannose substrates. These pathways converge at the point where complement component C3 is cleaved by an active protease (which is different in each pathway) to yield C3a and C3b. Other pathways activating complement attack can act later in the sequence of events leading to various aspects of complement function.

C3a is an anaphylatoxin. C3b binds to bacterial and other cells, as well as to certain viruses and immune complexes, and tags them for removal from the circulation. (C3b in this role is known as opsonin.) The opsonic function of C3b is generally considered to be the most important anti-infective action of the complement system. Patients with genetic lesions that block C3b function are prone to infection by a broad variety of pathogenic organisms, while patients with lesions later in the complement cascade sequence, i.e., patients with lesions that block C5 functions, are found to be more prone only to Neisseria infection, and then only somewhat more prone.

C3b also forms a complex with other components unique to each pathway to form classical or alternative C5 convertase, which cleaves C5 into C5a and C5b. C3 is thus regarded as the central protein in the complement reaction sequence since it is essential to both the alternative and classical pathways. This property of C3b is regulated by the serum protease Factor I, which acts on C3b to produce iC3b. While still functional as opsonin, iC3b cannot form an active C5 convertase.

C5 is a 190 kDa beta globulin found in normal serum at approximately 75 µg/ml (0.4 µM). C5 is glycosylated, with about 1.5-3 percent of its mass attributed to carbohydrate. Mature C5 is a heterodimer of a 999 amino acid 115 kDa alpha chain that is disulfide linked to a 656 amino acid 75 kDa beta chain. C5 is synthesized as a single chain precursor protein product of a single copy gene (Haviland et al. J. Immunol. 1991, 146:362-368). The cDNA sequence of the transcript of this gene predicts a secreted pro-C5 precursor of 1659 amino acids along with an 18 amino acid leader sequence (see, U.S. patent 6,355,245).

The pro-C5 precursor is cleaved after amino acid 655 and 659, to yield the beta chain as an amino terminal fragment (amino acid residues +1 to 655 of the above sequence) and the alpha chain as a carboxyl terminal fragment (amino acid residues 660 to 1658 of the above sequence), with four amino acids (amino acid residues 656-659 of the above sequence) deleted between the two.

C5a is cleaved from the alpha chain of C5 by either alternative or classical C5 convertase as an amino terminal fragment comprising the first 74 amino acids of the alpha chain (i.e., amino acid residues 660-733 of the above sequence). Approximately 20 percent of the 11 kDa mass of C5a is attributed to carbohydrate. The cleavage site for convertase action is at, or immediately adjacent to, amino acid residue 733 of the above sequence. A compound that would bind at, or adjacent, to this cleavage site would have the potential to block access of the C5 convertase enzymes to the cleavage site and thereby act as a complement inhibitor.

C5 can also be activated by means other than C5 convertase activity. Limited trypsin digestion (Minta and Man, J. Immunol. 1977, 119:1597-1602; Wetsel and Kolb, J. Immunol. 1982, 128:2209-2216) and acid treatment (Yammamoto and Gewurz, J. Immunol. 1978, 120:2008; Damerau et al., Molec. Immunol. 1989, 26:1133-1142) can also cleave C5 and produce active C5b.

Cleavage of C5 releases C5a, a potent anaphylatoxin and chemotactic factor, and leads to the formation of the lytic terminal complement complex, C5b-9. C5a and C5b-9 also have pleiotropic cell activating properties, by amplifying the release of downstream inflammatory factors, such as hydrolytic enzymes, reactive oxygen species arachidonic acid metabolites and various cytokines.

C5b combines with C6, C7, and C8 to form the C5b-8 complex at the surface of the target cell. Upon binding of several C9 molecules, the membrane attack complex (MAC, C5b-9, terminal complement complex--TCC) is formed. When sufficient numbers of MACs insert into target cell membranes the openings they create (MAC pores) mediate rapid osmotic lysis of the target cells. Lower, non-lytic concentrations of MACs can produce other effects. In particular, membrane insertion of small numbers of the C5b-9 complexes into endothelial cells and platelets can cause deleterious cell activation. In some cases activation may precede cell lysis.

As mentioned above, C3a and C5a are anaphylatoxins. These activated complement components can trigger mast cell degranulation, which releases histamine from basophils and mast cells, and other mediators of inflammation, resulting in smooth muscle contraction, increased vascular permeability, leukocyte activation, and other inflammatory phenomena including cellular proliferation resulting in hypercellularity. C5a also functions as a chemotactic peptide that serves to attract pro-inflammatory granulocytes to the site of complement activation.

C5a receptors are found on the surfaces of bronchial and alveolar epithelial cells and bronchial smooth muscles cells. C5a receptors have also been found on eosinophils, mast cells, monocytes, neutrophils, and activated lymphocytes. Thus, compounds that block engagement of receptors of complement components are useful herein.

Any compounds which bind to or otherwise block the generation and/or activity of any of the human complement components, such as, for example, antibodies specific to a human complement component are useful herein. Some compounds include antibodies directed against complement components C-1, C-2, C-3, C-4, C-5, C-6, C-7, C-8, C-9, Factor D, Factor B, Factor P, MBL, MASP-1, AND MASP-2, thus preventing the generation of the anaphylatoxic activity associated with C5a and preventing the assembly of the membrane attack complex associated with C5b. Also useful in the methods described herein are naturally occurring or soluble forms of complement inhibitory compounds such as CR1, LEX-CR1, MCP, DAF, CD59, Factor H, cobra venom factor, FUT-175, y bind protein, complestatin, and K76 COOH.

Particularly useful compounds for use as described herein are antibodies that reduce, directly or indirectly, the conversion of complement component C5 into complement components C5a and C5b. One class of useful antibodies are those having at least one antibody-antigen binding site and exhibiting specific binding to human complement component C5, wherein the specific binding is targeted to the alpha chain of human complement component C5. More particularly, a monoclonal antibody (mAb) may be used. Such an antibody 1) inhibits complement activation in a human body fluid; 2) inhibits the binding of purified human complement component C5 to either human complement component C3 or human complement component C4; and 3) does not specifically bind to the human complement activation product for C5a. Particularly useful complement inhibitors are compounds which reduce the generation of C5a and/or C5b-9 by greater than about 30%. Anti-C5 antibodies that have the desirable ability to block the generation of C5a have been known in the art since at least 1982 (Moongkarndi et al. Immunobiol. 1982, 162:397; Moongkarndi et al. Immunobiol. 1983, 165:323). Antibodies known in the art that are immunoreactive against C5 or C5 fragments include antibodies against the C5 beta chain (Moongkarndi et al. Immunobiol. 1982, 162:397; Moongkarndi et al. Immunobiol. 1983, 165:323; Wurzner et al. 1991, supra; Mollnes et al. Scand. J. Immunol. 1988, 28:307-312); C5a (see for example, Ames et al. J. Immunol. 1994,152:4572-4581, U.S. Pat. No. 4,686,100, and European patent publication No. 0 411 306); and antibodies against non-human C5 (see for example, Giclas et al. J. Immunol. Meth. 1987, 105:201-209). Particularly useful anti-C5 antibodies are h5G1.1, h5G1.1-scFv and functional fragments of h5G1.1. Methods for the preparation of h5G1.1, h5G1.1-scFv and functional fragments of h5G1.1 are described in U.S. Patent No. 6,355,245 and "Inhibition of Complement Activity by Humanized Anti-C5 Antibody and Single Chain Fv", Thomas et al, Molecular Immunology, Vol. 33, No. 17/18, pages 1389-1401, 1996.

Functionally, a suitable antibody inhibits the cleavage of C5, which blocks the generation of potent proinflammatory molecules C5a and C5b-9 (terminal complement complex). Preferably, the antibody does not prevent the formation of C3b, which subserves critical immunoprotective functions of opsonization and immune complex clearance.

While preventing the generation of these proinfammatory terminal complement components, antibody-mediated inhibition of the complement cascade at C5 preserves the ability to generate C3b, which is critical for opsonization of many pathogenic microorganisms, as well as for immune complex solubilization and clearance. Retaining the capacity to generate C3b appears to be particularly important as a therapeutic factor in complement inhibition for inflammatory diseases, where increased susceptibility to infection and impaired clearance of immune complexes are preexisting clinical features of the disease process.

The anti-human C5 antibody described herein is preferably a monoclonal antibody, although polyclonal antibodies produced and screened by conventional techniques can also be used if desired.

The preferred anti-C5 antibodies used to treat asthma in accordance with this disclosure bind to C5 or fragments thereof, e.g., C5a or C5b. Preferably, the anti-C5 antibodies are immunoreactive against epitopes on the alpha chain of purified human complement component C5 and are capable of blocking the conversion of C5 into C5a and C5b by C5 convertase. This capability can be measured using the techniques described in Wurzner, et al., Complement Inflamm 8:328-340, 1991.

In a particularly useful disclosure, the anti-C5 antibodies are not immunoreactive against epitopes on the beta chain, but rather are immunoreactive against epitopes within the alpha chain of purified human complement component C5. In this disclosure, the antibodies are also capable of blocking the conversion of C5 into C5a and C5b by C5 convertase. Within the alpha chain, the most preferred antibodies bind to an amino-terminal region, however, they do not bind to free C5a.

Hybridomas producing monoclonal antibodies reactive with complement component C5 can be obtained according to the teachings of Sims, et al., U.S. Pat. No. 5,135,916. Antibodies are prepared using purified components of the complement membrane attack complex as immunogens according to known methods. In accordance with this disclosure, complement component C5 or C5b is preferably used as the immunogen. In accordance with particularly preferred useful disclosures, the immunogen is the alpha chain of C₅.

Particularly useful antibodies share the required functional properties discussed in the preceding paragraph and have any of the following characteristics:
(1) they compete for binding to portions of C5-the C5 alpha chain; and
(2) they specifically bind to the C5 alpha chain. Such specific binding, and competition for binding can be determined by various methods well known in the art, including the plasmon surface resonance method (Johne et al., J. Immunol. Meth. 1993, 160:191-198).
(3) they block the binding of C5 to either C3 or C4 (which are components of C5 convertase).

A method of reducing inflammation in the lungs of asthma patients is provided. The methods include the step of administering to a subject having or susceptible to asthma a compound that reduces the production or release of inflammatory mediators in the airway of the subject. Non-limiting examples of inflammatory mediators that can be reduced in accordance with this disclosure include TGFβ, eosinophil granule proteins, and matrix metalloprotease 9 (mmp9 - also known as the 92-kDa type IV collagenase/gelatinase or gelatinase B). The compounds can reduce inflammation by any variety of mechanisms. Where the inflammatory mediator is MMP-9, for example, the compound can act at the cellular level to reduce the production or release of pro-mmp-9, can interact with pro-mmp-9 in a manner that prevents conversion of pro-mmp-9 to the 83 kDa active form or can interact with the active form of mmp9 to prevent the inflammatory effects associated with the enzyme (such as, for example, the generation of TGF-beta). Suitable compounds include, but are not limited to, the compounds described above which bind to or otherwise block the generation and/or release an/or activity of one or more complement components or block engagement of the receptors of complement components.

MMP-9 activity can be detected in accordance with a variety of art-recognized procedures. For example, quantitative zymographic methods provide a relatively refined assessment of the activity of this enzyme. This method allows for the detection of MMP-9 activity based upon the ability of the enzyme to hydrolyze denatured collagen, i.e., gelatin, which is a natural substrate for MMP-9. The gelatin is incorporated into a gel such as polyacrylamide. See Hibbs et al., J. Biol. Chem. 260:2493-2500 (1985) and Moll et al., Cancer Res. 50:6162-70 (1990). The assay may be standardized using a purified MMP-9 preparation that is analyzed in parallel with the test sample. Purified MMP-9 can be prepared by methods known in the art. See, for example, Okada et al., supra., and Morodomi et al., Biochem J. 285:603:11 (1992). The extent of hydrolysis of the gelatin is directly related to the activity of MMP-9 in the sample, and the active MMP-9 forms can be identified by their characteristic molecular weights. In the gelatin zymography, the proMMP-9 species can be detected because of the catalytic activation that occurs during electrophoresis and subsequent incubation. However, the MMP-9 forms present prior to the onset of labor are incapable of undergoing this kind of activation, i.e., they are latent.

MMP-9 activity can also be detected using standard immunological techniques, e.g., ELISA, immunofluorescence assays, or radioimmunoassays. In a preferred embodiment, MMP-9 activity is detected using ELISA, which entails the use of antibodies specific to MMP-9. See David et al, U.S. Pat. No. 4,376,110 (and references cited therein). Monoclonal antibodies specific to MMP-9 have been prepared using partially purified enzyme preparations. See, e,g., Moll et al., supra; Ramos-DeSimone et al., HYBRIDOMA 12(4):349-63 (1993) and Goldberg et al. Polyclonal antibodies specific to MMP-9 can also be prepared in accordance with standard procedures. In a preferred disclosure, polyclonal antibodies are prepared using non-conserved peptides conjugated to a macromolecular carrier. The choice of a specific non-conserved peptide such as the metal-binding domain, among the members of the MMPs is considered within a level of ordinary skill in the art. See Woessner, and Goldberg et al., supra. Enzymic assays that can detect MMP-9 in picogram or nanogram amounts are also disclosed in Manicourt et al., Anal. Biochem. 215(2):171-9 (1993).

MMP-9 activity can further be detected in a sample by western blot analysis, which requires electrophoretic separation of the test material in a gel, followed by transfer of the separated proteins to a nitrocellulose membrane and detection of the MMP-9 antigens with a specific antibody and reagent that reacts with the antigen-fixed antibody. See Towbin et al., Proc. Natl. Acad. Sci. USA 76(9):4350-4354 (1979).

Suitable assays for detection of mmp9 are commercially available from a variety of sources such as, for example Boehringer Manheim Biochemicals (Manheim, Germany) and R&D Systems, (Minneapolis, MN).

The compound that inhibits the production and/or activity of at least one complement component can be administered in a variety of unit dosage forms. The dose will vary according to the particular compound employed. For example, different antibodies may have different masses and/or affinities, and thus require different dosage levels. Antibodies prepared as Fab' fragments will also require differing dosages than the equivalent intact immunoglobulins, as they are of considerably smaller mass than intact immunoglobulins, and thus require lower dosages to reach the same molar levels in the patient's blood.

The dose will also vary depending on the manner of administration, the particular symptoms of the patient being treated, the overall health, condition, size, and age of the patient, and the judgment of the prescribing physician.

Administration of the compound that inhibits the production and/or activity of at least one complement component will generally be in an aerosol form with a suitable pharmaceutical carrier, via intravenous infusion by injection, or subcutaneous injection. Other routes of administration may be used if desired. Aerosol administration is preferred since it avoids the systemic effects of the complement-inhibiting compound, while providing the desired asthma-treating effects to be achieved in accordance with this disclosure.

Formulations suitable for injection are found in Remington's Pharmaceutical Sciences, Mack Publishing Company, Philadelphia, Pa., 17th ed. (1985). Such formulations must be sterile and non-pyrogenic, and generally will include a pharmaceutically effective carrier, such as saline, buffered (e.g., phosphate buffered) saline, Hank's solution, Ringer's solution, dextrose/saline, glucose solutions, and the like. The formulations may contain pharmaceutically acceptable auxiliary substances as required, such as, tonicity adjusting agents, wetting agents, bactericidal agents, preservatives, stabilizers, and the like.

### EXAMPLE 1-The Use Of Anti-C5 Antibodies As A Prophylactic For C5 Inhibition To Treat Asthma.

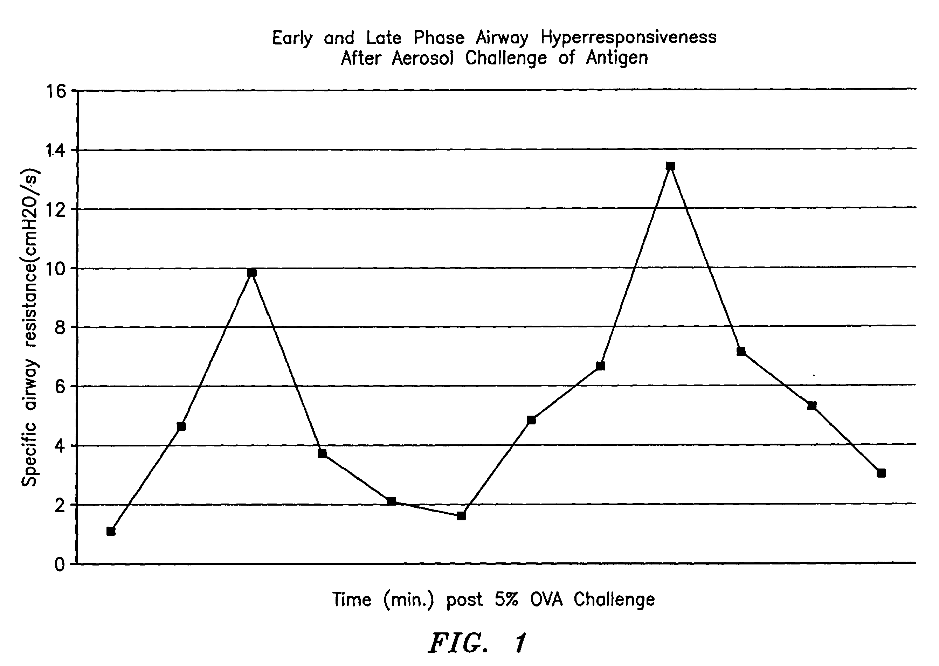

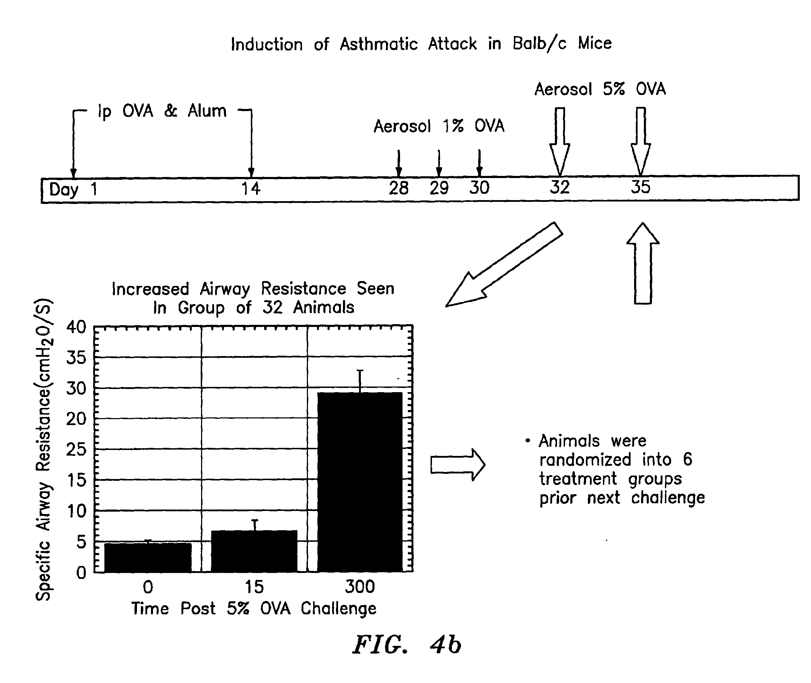

Asthma was induced in normal BALB/c mice by exposing them to Ovalbumin antigen ("OVA") and Alum, according to the dosages and schedule shown in Figure 4b. These exposures solicited the standard asthmatic response during an attack. The exposed mice showed both early phase and late phase reactions. As seen in Figure 1, the early phase reaction was specific airway resistance within 15 minutes after exposure. A more severe late phase reaction of specific airway resistance occurred approximately 5 hours after exposure. Specific airway resistance was measured by means of double chamber plethysmograph fitted with pneumotachographs, which is commercially available from the Buxo Corporation.

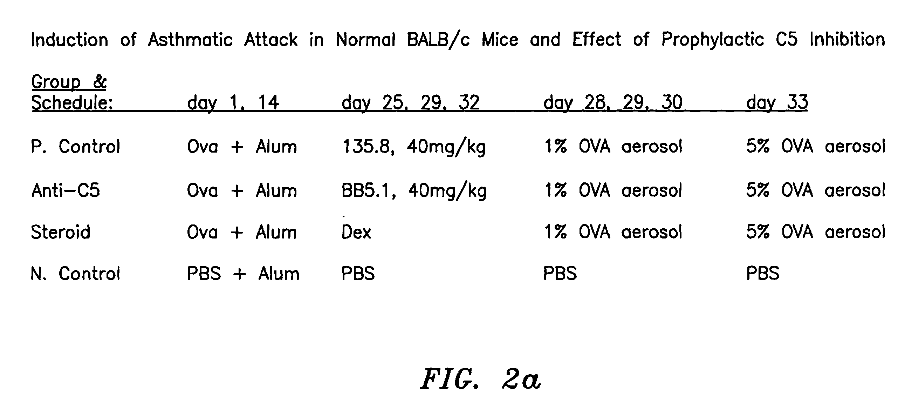

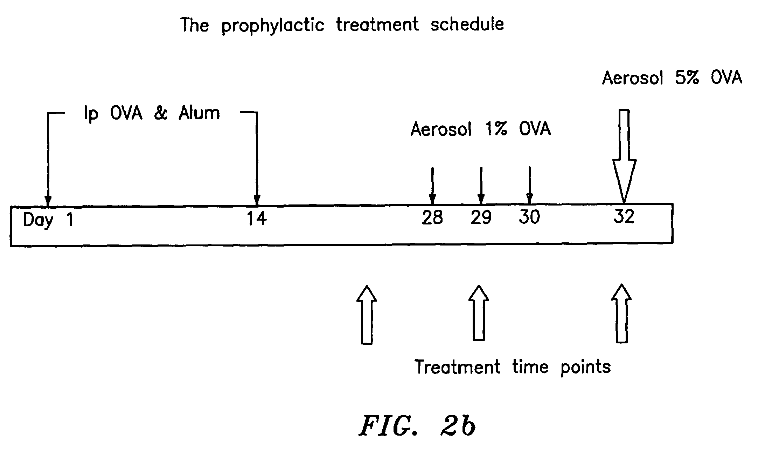

To demonstrate the prophylactic benefits of a complement component inhibiting compound, groups of mice were given one of three different treatments in accordance with the schedule of dosages shown in Figures 2a and 2b. The positive control group was treated with a control antibody (hybridoma 135.8) at a dose of 40 mg/kg. A second group of mice was treated with the anti-C5 antibody, BB5.1, at a dose of 40mg/kg. A third group of mice was treated with Dexamethasone ("DEX"). A negative control group was initially exposed to PBS (phosphate buffer saline) and Alum, and was later given PBS as shown in the schedule of Figure 2a. This control group provided a baseline air resistance measurement.

The BB5.1 antibody is made according to known methods. (See, Frei, Y., Lambris, J.D., Stockinger, B. Mol. Cell. Probes. 1: 141-149 (1987)). Both the BB5.1 antibody and the isotype match control 135.8 hybridoma antibody were grown as ascites in athymic mice and the antibodies were purified from ascites by protein A affinity chromatography followed by elution with ImmunoPure IgG elution buffer (Pierce) and dialysis against PBS buffered saline. (Wang et al. 1996).

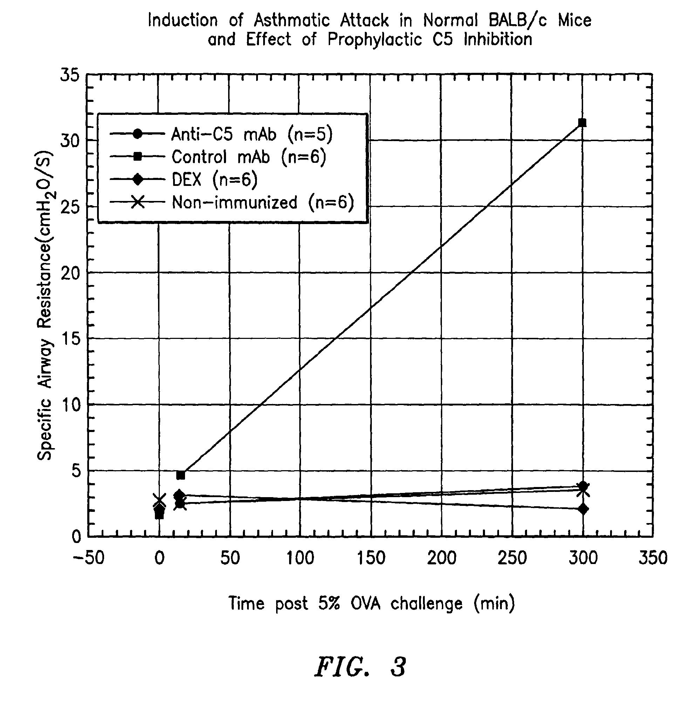

It was found that the group prophylactically treated subcutaneously with Anti-C5 antibody responded as well as the challenged mice treated with steroids and the negative control, as shown in Figure 3. The Anti-C5 treatments had inhibited the production of complement component C5 in the cascade, and by inhibiting C5 production, the mice experienced significantly less airway constriction. The positive control group that was treated with a control antibody showed increased specific airway resistance caused by an asthmatic attack.

### EXAMPLE 2 - The Use Of Anti-C5 Antibodies Via Intravenous Administration As A Therapeutic Method To Treat Asthma

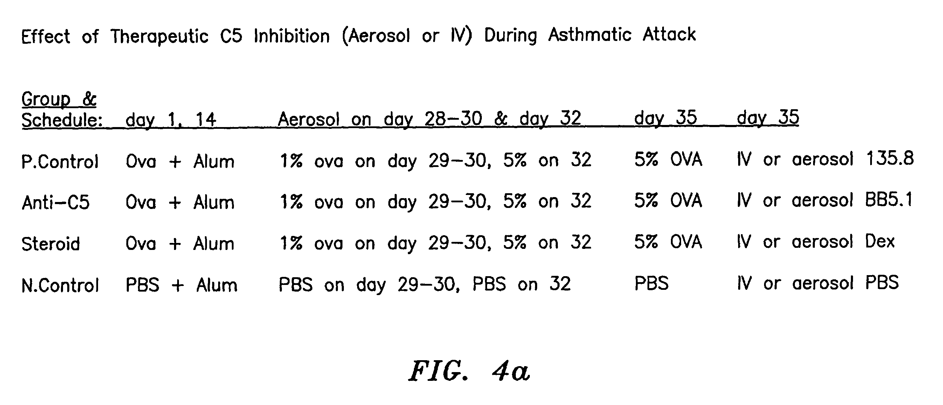

To demonstrate the therapeutic benefits of administering a C5 complement component inhibitor intravenously during an asthma attack, groups of mice were given one of three different treatments in accordance with the schedule of dosages shown in Figure 4a. A positive control group was challenged with OVA and treated with a control antibody (135.8 hybridoma) administered intravenously, 15 minutes after provoking the initial asthmatic attack. A second group of mice were similarly challenged and intravenously treated with BB5.1, the anti-C5 antibody, 15 minutes after provoking the initial asthmatic attack. A third group of mice were similarly challenged and intravenously treated with DEX, 15 minutes after provoking the initial asthmatic attack. Sham operated mice provided baseline air resistance measurement which was provided by administering phosphorate buffer solution (PBS) and alum to a group of mice, and administering aerosol doses of PBS according to the schedule shown on Fig. 4a.

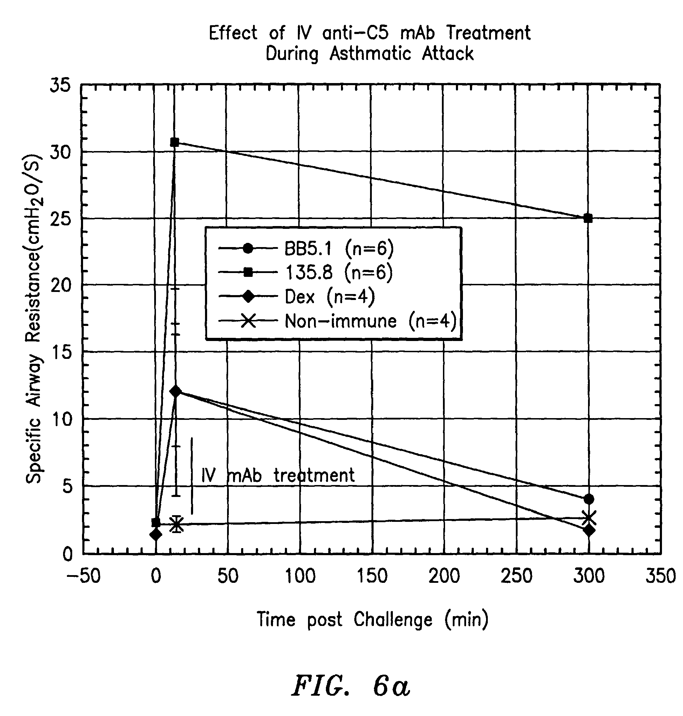

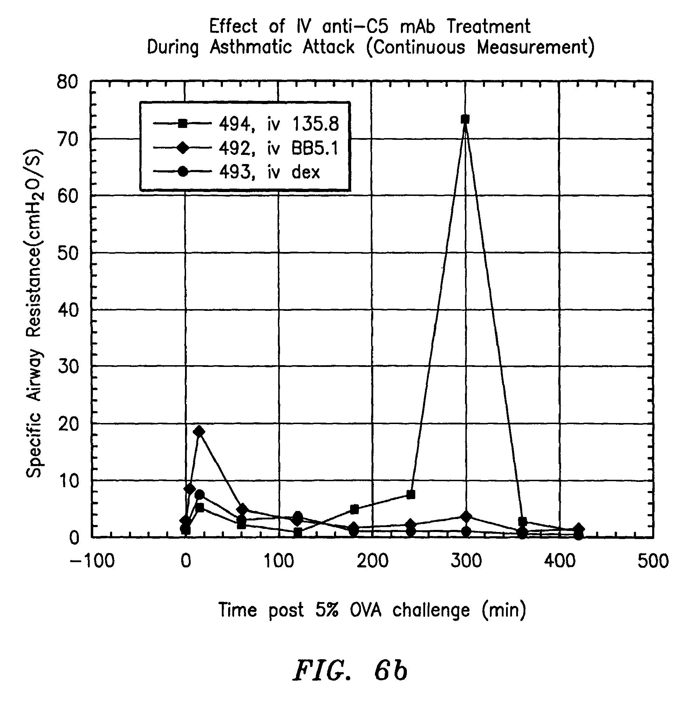

The results of this challenge (shown in Figs. 6a and 6b) found that the group of mice treated therapeutically with anti-C5 intravenously showed very little specific airway constriction and these mice responded to the treatments as well as the group treated with the steroid, DEX. The positive control group that was treated with a control antibody showed significantly increased specific airway resistance, as shown in Figure 6a. The anti-C5 antibody had inhibited the inflammatory response of the complement components, and allowed greater air passage during the asthma attacks in the mice.

### EXAMPLE 3 - The Use Of Anti-C5 Antibodies Via Aerosol Administration As A Therapeutic Method To Treat Asthma

To demonstrate the therapeutic benefits of administering a C5 complement component inhibitor via aerosol during an asthma attack, groups of mice were given one of three different treatments in accordance with the schedule of dosages shown in Figure 4a. A positive control group was challenged with OVA and treated with a control antibody (135.8 hybridoma) administered by aerosol, 15 minutes after provoking the initial asthmatic attack. A second group of mice were similarly challenged and treated via aerosol administration of BB5.1, an anti-C5 antibody, 15 minutes after provoking the initial asthmatic attack. A third group of mice were similarly challenged and treated by aerosol with DEX, 15 minutes after provoking the initial asthmatic attack. A baseline air resistance measurement was provided by administering phosphorate buffer solution (PBS) and alum to a group of mice, and administering aerosol doses of PBS according to the schedule shown on Fig. 4a.

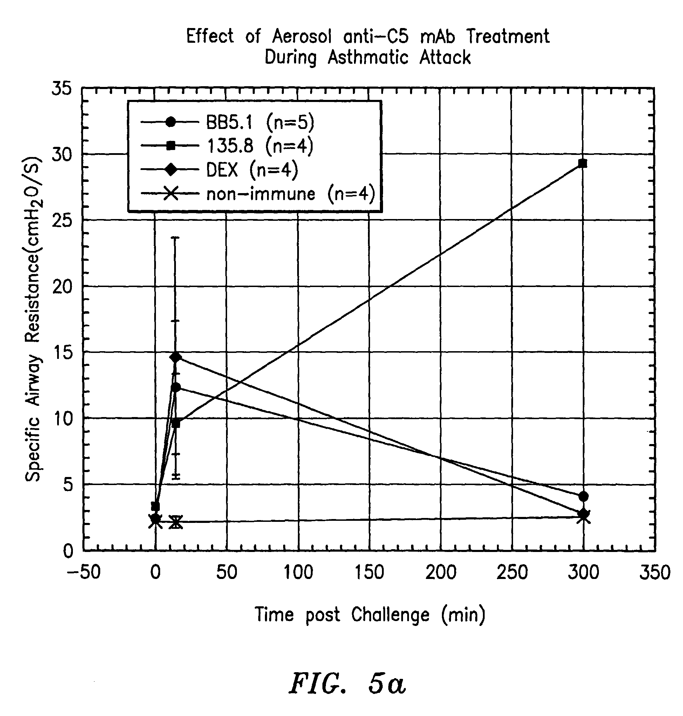

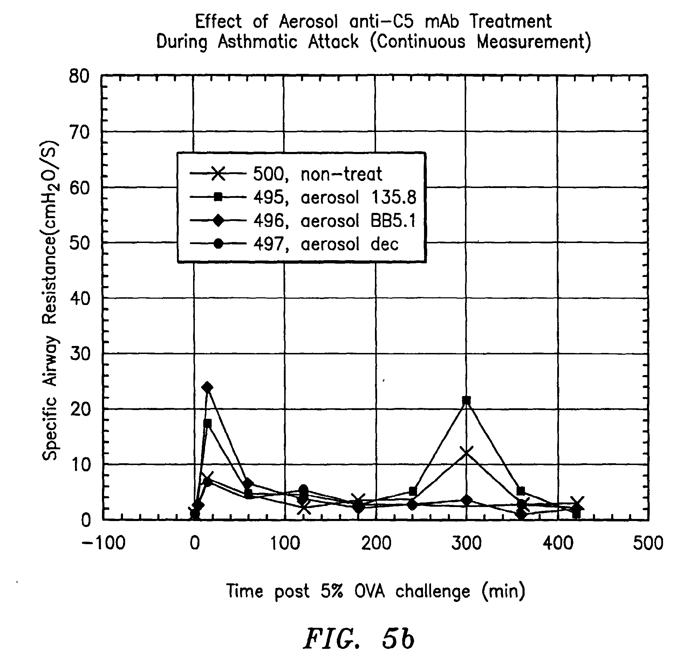

It was found that therapeutic aerosol treatments of mice with anti-C5 antibody, BB5.1 during asthma attacks significantly lowered the specific airway resistance and these mice responded as well as, and more quickly than, the steroid treated mice. The results of the aerosol treatments are shown in Figures 5a and 5b. The positive control group that was treated with a control antibody experienced specific airway resistance many times greater than the mice treated with anti-C5 antibody, as shown in Figure 5b.

Anti-C5 antibody, BB5.1 was also administered to the test animals through nebulization, which was found to be an effective method of administration. Results of serum tests after nebulization indicate that the BB5.1 was binding with the C5 site and, therefore, remained intact during delivery through nebulization.

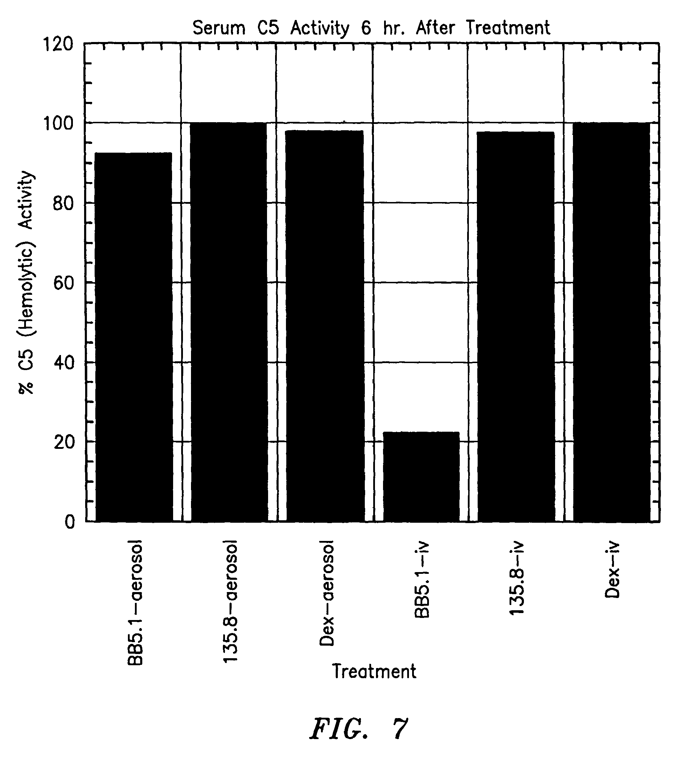

The systemic effect of each treatment given in Examples 2 and 3 was measured using the techniques described in Wurzner, et al., Complement Inflamm 8:328-340, 1991 for hemolytic activity. The results of these tests are shown in Figure 7. As seen therein, the control antibody and steroid did not substantially reduce systemic C5 activity, irrespective of the method of administration. With the anti-C5 antibody BB5.1, however, the manner of administration directly affected systemic C5 activity. Specifically, although both aerosol and intravenous administration were effective at reducing the severity of an asthma attack, the aerosol administration did so without substantially reducing systemic C5 activity. As seen in Figure 7, intravenous administration of the anti-C5 antibody reduced systemic C5 activity by nearly 80%.

### EXAMPLE 4 - The Use Of Anti-C5 Antibodies To Reduce the Presence of Inflammatory mediators

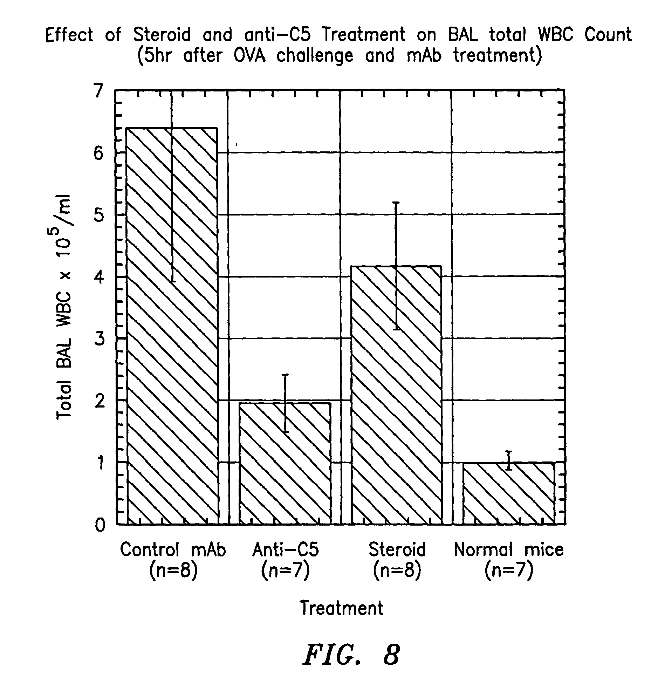

To demonstrate the therapeutic benefits of administering a C5 complement component inhibitor in reducing the presence of inflammatory mediators during an asthma attack, groups of mice were given one of three different treatments in accordance with the schedule of dosages shown in Figure 4a. A positive control group was challenged with OVA and treated with a control antibody (135.8 hybridoma) administered intravenously, 15 minutes after provoking the initial asthmatic attack. A second group of mice were similarly challenged and intravenously treated with BB5.1, the anti-C5 antibody, 15 minutes after provoking the initial asthmatic attack. A third group of mice were similarly challenged and intravenously treated with DEX, 15 minutes after provoking the initial asthmatic attack. Sham operated mice provided baseline air resistance measurement which was provided by administering phosphorate buffer solution (PBS) and alum to a group of mice, and administering aerosol doses of PBS according to the schedule shown on Fig. 4a. After 5 hours the mice were euthanized and the lungs were lavaged using conventional techniques. Generally, 1 cc of PBI saline was introduced into the lungs and recovered. This process was repeated three times. The fluid was recovered and centrifuged. The cells contained in the resulting pellet were inspected. The supernatant was tested for the presence of histamine, IL-5, IL-4, IL-13, Eosinophils granule proteins, TGFβ and/or mmp-9. The assays were all conducted using commercially available test kits. The results are shown in Figures 8 through 13. These BronchoAlveolarLavage (*BAL*) results show the presence or absence of proteins which were produced or released by lung structure cells or inflammatory cells, rather than the presence of such components in the lung tissue itself.

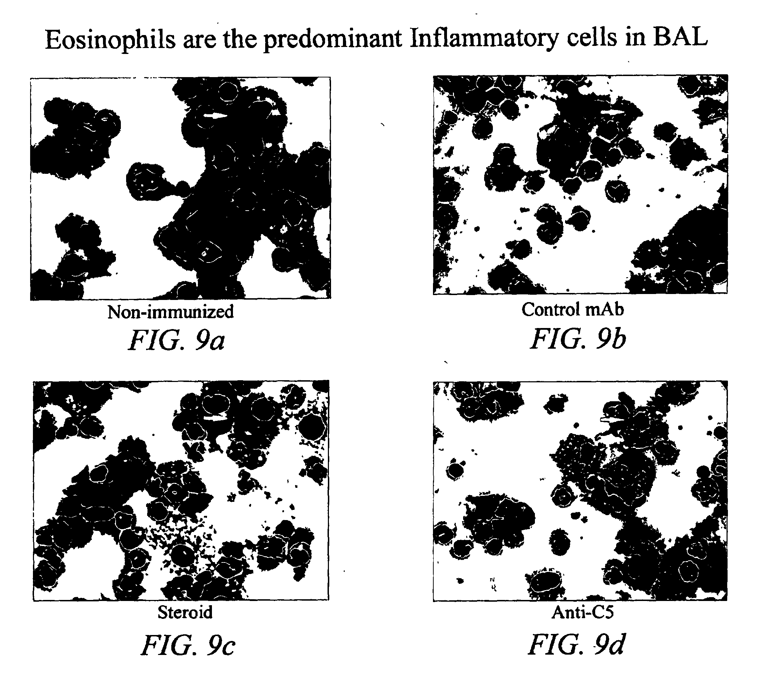

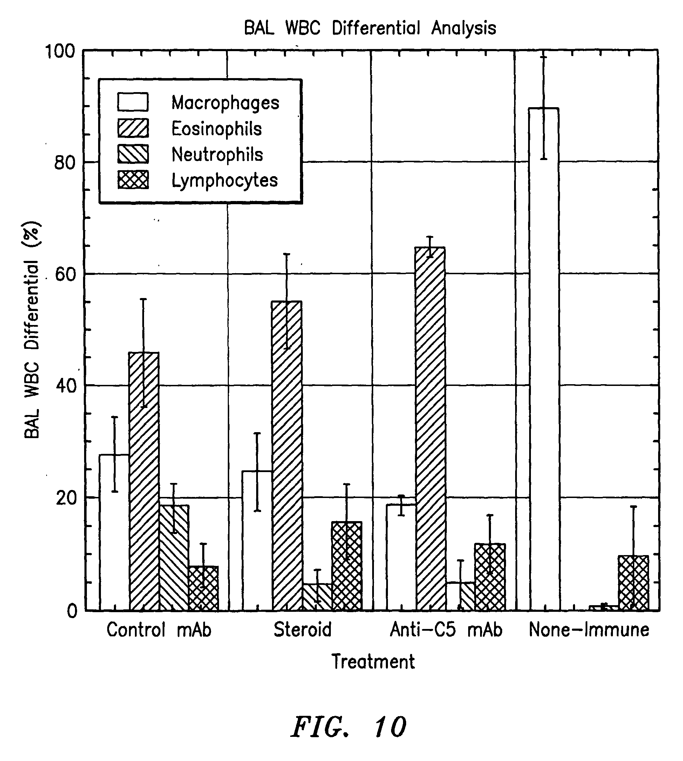

It was found that treatment of mice with anti-C5 antibody and DEX both reduced the total WBC count. (See Fig. 8). Eosinophils were found to be the predominant inflammatory cells in the BAL. (See Figs. 9A-D and 10.)

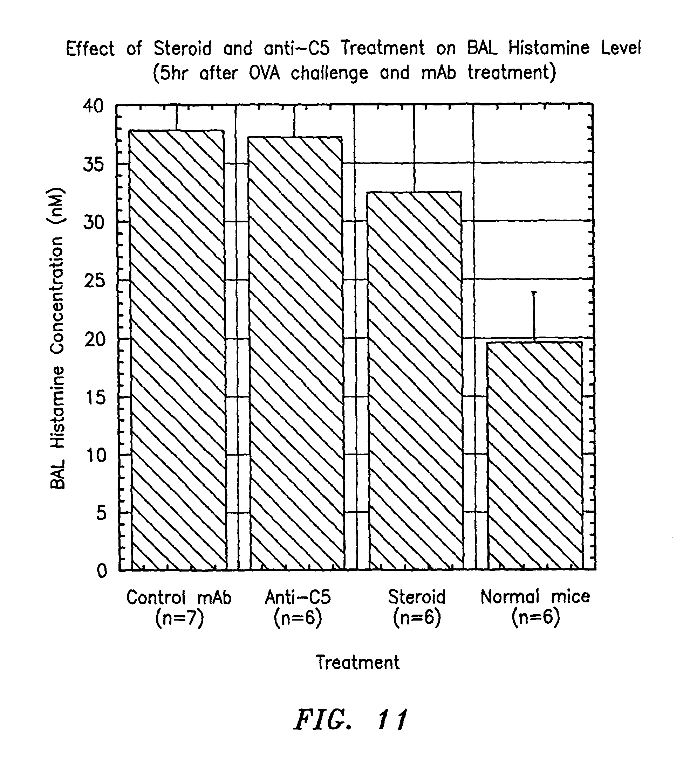

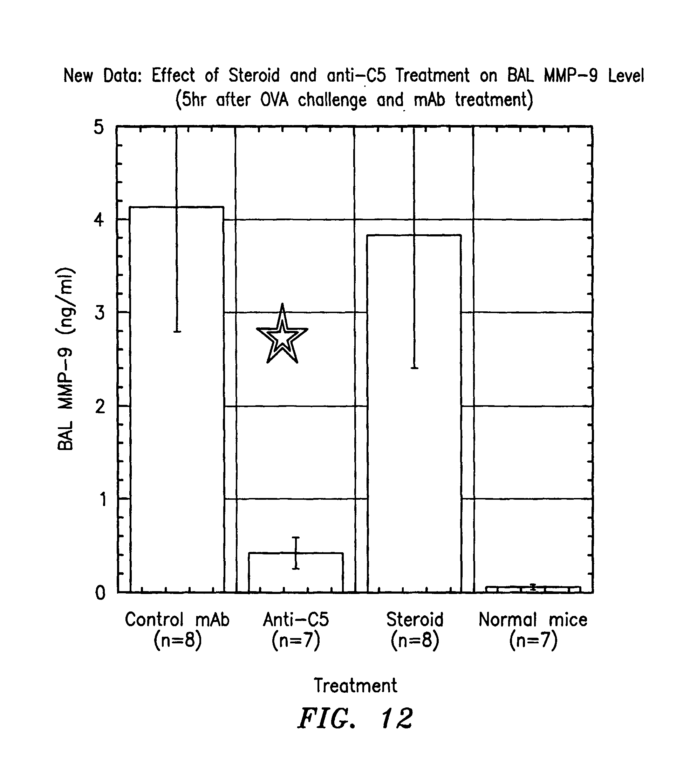

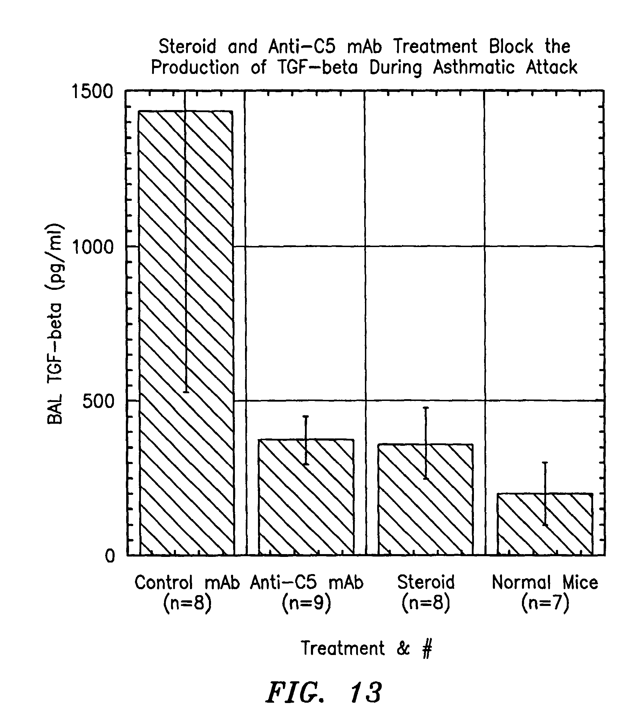

Figure 11 shows that the anti-C5 antibody had little effect on histamine level, a result similar to that obtained with the steroid DEX. However, the anti-C5 antibody significantly reduced the level of mmp-9 detected in BAL as shown in Figure 12, compared to treatment with Steroid. Thus, while both anti-C5 antibody and DEX resulted in a lower detectable amount of TGF-β (see Fig. 13), only the anti-C5 antibody did so through a mechanism that involved a reduction in production or release of mmp-9.

### EXAMPLE 5 - The Use Of Anti-C5 Antibodies As Bronchial Dilator

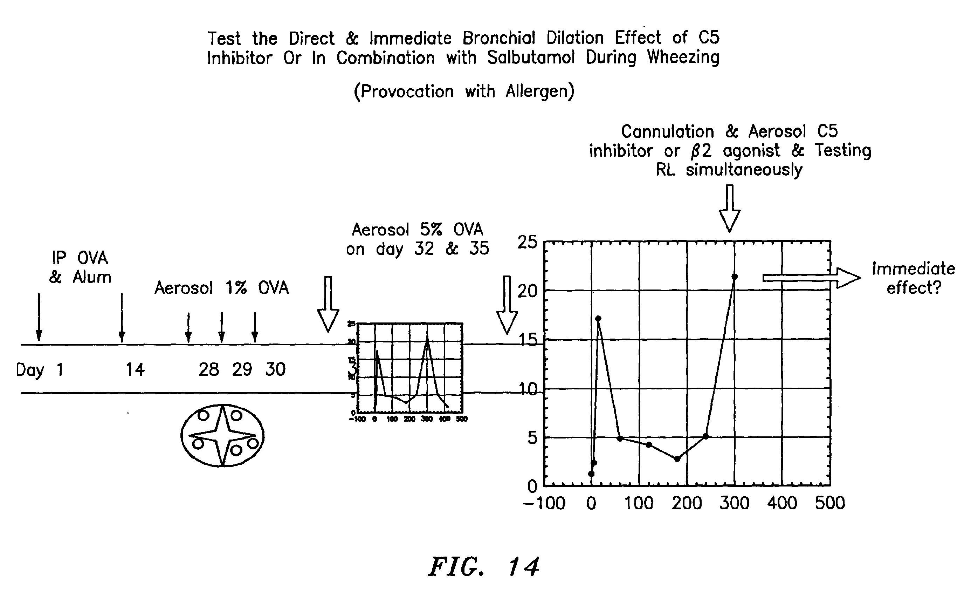

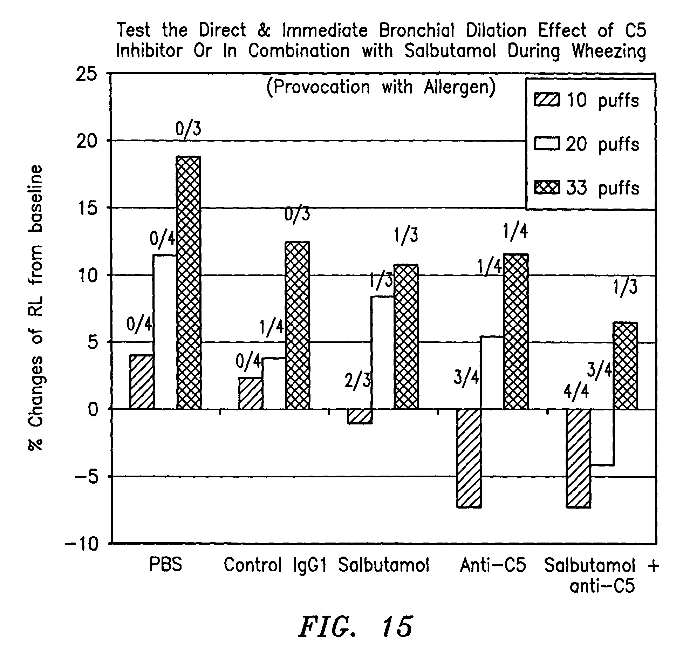

To demonstrate the direct and immediate bronchial dilation effect of administering a C5 complement component inhibitor (alone or in combination with a β2 adreno receptor agonist), during an asthma attack, groups of mice were challenged with antigen and given one of four different treatments in accordance with the schedule of dosages shown in Figures 14 and 15. A positive control group was challenged with OVA, cannulated and treated with a control antibody (mouse IgG1) administered via aerosol after provoking a second asthmatic attack. A second group of mice were similarly challenged and treated with BB5.1, an anti-C5 antibody after provoking a second asthmatic attack. A third group of mice were similarly challenged and treated with salbutamol (a β2 adreno receptor agonist commercially available from Sigma) after provoking a second asthmatic attack. A fourth group of mice were similarly challenged and treated with a combination of anti-C5 antibody and salbutamol after provoking a second asthmatic attack. Sham operated mice provided baseline air resistance measurement which was provided by administering phosphorate buffer solution (PBS) and alum to a group of mice, and administering aerosol doses of PBS according to the schedule shown on Figs. 14 and 15.

Airway responsiveness was then assessed as a change in airway function by an invasive method wherein changes in lung resistance were measured by using Buxco Biosystem software and whole body plethysmograph. Mice were anesthetized with Avertin (160mg/kg) by i.p. injection and ventilated by a Harvard Apparatus Inspira ventilator. After tracheal cannula, pancuronium (0.3mg/kg) was injected intraperitoneally to induce paralysis and inhibit spontaneous breathing. The mice are kept breathing by a ventilator, which is set at a tidal volume and respiratory rate by program of body weight. The measurements of RL to specific antigen were performed at 5 hours after 5% OVA provocation. The results, which are reported in Figure 15 show that the treatment with anti-C5 antibody had a significant bronchial dilation effect in 3 of 4 mice and that a synergistic effect is observed from the treatment with anti-C5 antibody in combination with a β2 adreno receptor agonist.

## Claims

1. An anti-C5 antibody for use in treating asthma in a subject susceptible to or having asthma, wherein the anti-C5 antibody is h5G1.1.

2. An anti-C5 antibody for use in reducing the severity of an asthma attack in a subject having an asthma attack, wherein the anti-C5 antibody is h5G1.1.

3. An anti-C5 antibody for use in reducing airway obstruction in a subject having asthma, wherein the anti-C5 antibody is h5G1.1.

4. The anti-C5 antibody or use according to any one of claims 1-3, wherein the use comprises administration of the anti-C5 antibody to the subject during an asthma attack.

5. The anti-C5 antibody for use according to any one of claims 1-4, wherein the subject is a human.

6. The anti-C5 antibody for use according to any one of claims 1-5, wherein the anti-C5 antibody is administered to the subject as an aerosol.

7. The anti-C5 antibody for use according to any one of claims 1-5, wherein the anti-C5 antibody is administered to the subject by intravenous infusion or subcutaneous injection.

8. The anti-C5 antibody for use according to any one of claims 1-7, wherein the use comprises administering to the subject a drug selected from the group consisting of steroids, anti-IgE antibodies, anti-IL-4 antibodies, anti-IL-5 antibodies, β2 adreno receptor agonists, leukotriene inhibitors, 5 lipoxygenase inhibitors, PDE inhibitors, CD23 antagonists, IL-13 antagonists, cytokine release inhibitors, histamine H1 receptor antagonists, anti-histamines, and histamine release inhibitors.

9. A drug and an anti-C5 antibody in combination for use in treating a subject susceptible to or having asthma, wherein the anti-C5 antibody is h5G1.1 and wherein the drug is selected from the group consisting of steroids, anti-IgE antibodies, anti-IL-4 antibodies, anti-IL-5 antibodies, β2 adreno receptor agonists, leukotriene inhibitors, 5 Lipoxygenase inhibitors, PDE inhibitors, CD23 antagonists, IL-13 antagonists, cytokine release inhibitors, histamine H1 receptor antagonists, anti-histamines, and histamine release inhibitors.

10. The anti-C5 antibody for use according to any one of claims 1-3, wherein the anti-C5 antibody is administered to the subject by nebulization.

## Patentansprüche

1. Anti-C5-Antikörper zur Verwendung bei der Behandlung von Asthma bei einem Individuum, das für Asthma anfällig ist oder daran leidet, wobei es sich bei dem Anti-C5-Antikörper um h5G1.1 handelt.

2. Anti-C5-Antikörper zur Verwendung beim Verringern der Schwere eines Asthmaanfalls bei einem Individuum, das einen Asthmaanfall erleidet, wobei es sich bei dem Anti-C5-Antikörper um h5G1.1 handelt.

3. Anti-C5-Antikörper zur Verwendung beim Verringern der Blockierung der Atemwege bei einem Individuum mit Asthma, wobei es sich bei dem Anti-C5-Antikörper um h5G1.1 handelt.

4. Anti-C5-Antikörper zur Verwendung nach einem der Ansprüche 1-3, wobei die Verwendung eine Verabreichung des Anti-C5-Antikörpers an das Individuum während eines Asthmaanfalls umfasst.

5. Anti-C5-Antikörper zur Verwendung nach einem der Ansprüche 1-4, wobei es sich bei dem Individuum um einen Menschen handelt.

6. Anti-C5-Antikörper zur Verwendung nach einem der Ansprüche 1-5, wobei der Anti-C5-Antikörper dem Individuum als Aerosol verabreicht wird.

7. Anti-C5-Antikörper zur Verwendung nach einem der Ansprüche 1-5, wobei der Anti-C5-Antikörper dem Individuum durch intravenöse Infusion oder subkutane Injektion verabreicht wird.

8. Anti-C5-Antikörper zur Verwendung nach einem der Ansprüche 1-7, wobei die Verwendung das Verabreichen eines aus der aus Steroiden, Anti-IgE-Antikörpern, Anti-IL-4-Antikörpern, Anti-IL-5-Antikörpern, β2-Adrenorezeptor-Agonisten, Leukotrien-Inhibitoren, 5-Lipoxygenase-Inhibitoren, PDE-Inhibitoren, CD23-Antagonisten, IL-13-Antagonisten, Cytokinfreisetzung-Inhibitoren, Histamin-H1-Rezeptor-Antagonisten, Antihistaminen und Histaminfreisetzung-Inhibitoren bestehenden Gruppe ausgewählten Arzneistoffs an das Individuum umfasst.

9. Arzneistoff und Anti-C5-Antikörper in Kombination zur Verwendung bei der Behandlung eines Individuums, das für Asthma anfällig ist oder daran leidet, wobei es sich bei dem Anti-C5-Antikörper um h5G1.1 handelt und wobei der Arzneistoff aus der aus Steroiden, Anti-IgE-Antikörpern, Anti-IL-4-Antikörpern, Anti-IL-5-Antikörpern, β2-Adrenorezeptor-Agonisten, Leukotrien-Inhibitoren, 5-Lipoxygenase-Inhibitoren, PDE-Inhibitoren, CD23-Antagonisten, IL-13-Antagonisten, Cytokinfreisetzung-Inhibitoren, Histamin-H1-Rezeptor-Antagonisten, Antihistaminen und Histaminfreisetzung-Inhibitoren bestehenden Gruppe ausgewählt ist.

10. Anti-C5-Antikörper zur Verwendung nach einem der Ansprüche 1-3, wobei der Anti-C5-Antikörper dem Individuum durch Vernebelung verabreicht wird.

## Revendications

1. Anticorps anti-C5 pour utilisation dans le traitement de l'asthme chez un sujet susceptible de ou ayant de l'asthme, l'anticorps anti-C5 étant h5G1.1.

2. Anticorps anti-C5 pour utilisation dans la réduction de la gravité d'une attaque d'asthme chez un sujet ayant une attaque d'asthme, l'anticorps anti-C5 étant h5G1.1.

3. Anticorps anti-C5 pour utilisation dans la réduction d'une obstruction des voies respiratoires chez un sujet ayant de l'asthme, l'anticorps anti-C5 étant h5G1.1.

4. Anticorps anti-C5 pour utilisation selon l'une quelconque des revendications 1 à 3, l'utilisation comprenant l'administration de l'anticorps anti-C5 au sujet pendant une attaque d'asthme.

5. Anticorps anti-C5 pour utilisation selon l'une quelconque des revendications 1 à 4, le sujet étant un humain.

6. Anticorps anti-C5 pour utilisation selon l'une quelconque des revendications 1 à 5, l'anticorps anti-C5 étant administré au sujet sous la forme d'un aérosol.

7. Anticorps anti-C5 pour utilisation selon l'une quelconque des revendications 1 à 5, l'anticorps anti-C5 étant administré au sujet par perfusion intraveineuse ou injection sous-cutanée.

8. Anticorps anti-C5 pour utilisation selon l'une quelconque des revendications 1 à 7, l'utilisation comprenant l'administration au sujet d'un médicament choisi dans le groupe constitué de stéroïdes, d'anticorps anti-IgE, d'anticorps anti-IL-4, d'anticorps anti-IL-5, d'agonistes de récepteur β2-adrénergique, d'inhibiteurs de leucotriène, d'inhibiteurs de 5-lipoxygénase, d'inhibiteurs de PDE, d'antagonistes de CD23, d'antagonistes de IL-13, d'inhibiteurs de libération de cytokine, d'antagonistes de récepteur d'histamine H1, d'antihistaminiques et d'inhibiteurs de libération d'histamine.

9. Médicament et anticorps anti-C5 en combinaison pour utilisation dans le traitement d'un sujet susceptible de ou ayant de l'asthme, l'anticorps anti-C5 étant h5G1.1 et le médicament étant choisi dans le groupe constitué de stéroïdes, d'anticorps anti-IgE, d'anticorps anti-IL-4, d'anticorps anti-IL-5, d'agonistes de récepteur β2-adrénergique, d'inhibiteurs de leucotriène, d'inhibiteurs de 5-lipoxygénase, d'inhibiteurs de PDE, d'antagonistes de CD23, d'antagonistes de IL-13, d'inhibiteurs de libération de cytokine, d'antagonistes de récepteur d'histamine H1, d'antihistaminiques et d'inhibiteurs de libération d'histamine.

10. Anticorps anti-C5 pour utilisation selon l'une quelconque des revendications 1 à 3, l'anticorps anti-C5 étant administré au sujet par nébulisation.
